# EUROPEAN PATENT SPECIFICATION

(11) **EP 1 397 192 B1**
(45) Date of publication and mention of the grant of the patent: **04.10.2006**
(21) Application number: 02748768.5
(22) Date of filing: 10.06.2002
(51) Int. Cl.: B01D 53/14, B01D 53/18, C07D 301/32

(54) **PROCESS FOR THE RECOVERY OF COMBUSTIBLE COMPONENTS OF A GAS STREAM**
VERFAHREN ZUR WIEDERGEWINNUNG VON BRENNBAREN BESTANDTEILEN AUS EINEM GASSTROM
TRAITEMENT DE RECUPERATION DES COMPOSANTS COMBUSTIBLES DANS UN COURANT GAZEUX

(30) Priority: 18.06.2001 EP 01114576
(43) Date of publication of application: 17.03.2004
(73) Proprietor: Degussa AG, 40474 Düsseldorf (DE); Uhde GmbH, 44141 Dortmund (DE)
(72) Inventor: HOFEN, Willi, 63517 Rodenbach (DE); THIELE, Georg, 63450 Hanau (DE); WOELL, Wolfgang, 63477 Maintal (DE)
(74) Representative: Sternagel, Fleischer, Godemeyer & Partner
(86) International application number: PCT/EP2002/006448
(87) International publication number: WO 2002/102496

(56) References cited:
- EP-A- 0 583 828
- EP-A- 0 719 768
- EP-A- 0 827 765

## Description

The present invention refers to a process for the recovery of combustible components of a gas stream comprising the combustible components and oxygen by selective absorption of the combustible components in a solvent. The present invention in particular refers to a process for recovery of combustible components from an exit gas stream from an oxidation reaction with a peroxide component, whereby due to the decomposition of the peroxide oxygen is accumulated during the oxidation reaction. The present invention is particularly suitable for a working-up stage in a process for epoxidation of olefins.

In numerous processes involving oxidation with peroxide compounds, particularly hydrogen peroxide, gas mixtures are formed that comprise considerable amounts of organic combustible components in addition to molecular oxygen resulting from the decomposition of the peroxide compounds. For safety reasons, these processes have to be conducted to ensure that the oxygen content is below the explosion limit. For economic reasons, it is often necessary to recover the combustible components of an exit gas stream, since they may contain valuable compounds like, for example, product compounds or components that can be recycled to the initial reaction stage. Therefore, it has been proposed to recover the combustible components by selective absorption in a suitable solvent.

The oxygen content of the gas phase increases within the absorption unit due to the absorption of organic combustible components in the solvent. This may have the consequence that even if the gas stream entering the absorption unit is not an ignitable composition, it may become ignitable during the process of absorption. Therefore, for safety reasons, an inert gas is introduced in the absorption unit to avoid, under any condition, the formation of an ignitable composition within the absorption unit.

For example, EP-A-719 768 describes a process for the epoxidation of an olefin with hydrogen peroxide, wherein a gas mixture of the olefin and oxygen from hydrogen peroxide decomposition is separated from the liquid epoxidation reaction mixture and the olefin is absorbed from the gas mixture in a liquid absorbent and inert gas is added to the oxygen in an amount sufficient to prevent formation of a flammable gas composition. Referring to the example in EP-A-719 768 the absorption liquid is introduced into the upper section and the gaseous purge stream into the lower section of the absorption zone to ensure a counter-current flow. But there is no indication whether the liquid phase or the gas phase is the continuous phase.

EP-A-583 828 discloses a process for ethylene recovery in a process of direct oxidation of ethylene oxide. According to this process the direct-oxidation product is worked up in three different stages. In the third stage unreacted ethylene is removed by absorption using a high molecular weight organic liquid for example paraffins. From the information given for performing the absorption it is not evident that the gas phase is dispersed in a continuous liquid phase. On the contrary the information that the contacting surface may be created by trays, structured packing or random dump packing implies to the person skilled in the art that the gas phase is the continuous phase and the liquid phase is the dispersed phase since otherwise the contacting surface between gas and liquid phase cannot be increased using packings in the absorber column.

Similarly, European Patent Application 00102542.8 discloses a process for the working-up of an exit gas stream from the epoxidation of an olefin with hydrogen peroxide, comprising olefin oxide, unreacted olefin, and oxygen, whereby the exit gas stream is brought into contact in an absorption unit with the same solvent as used in the epoxidation stage, and a solvent stream loaded with olefin and olefin oxide is drawn off from the absorption unit, and an exit gas stream containing oxygen is discharged. Additionally, an inert gas stream is introduced into the absorption unit, wherein the inert gas leaves the absorption unit together with the oxygen in the exit gas stream. The quantity of inert gas introduced is preferably selected as a function of the quantity and composition of the exit gas stream leaving the reaction stage, such that the exit gas stream leaving the absorption unit is no longer an ignitable composition. Thereby, measures are taken to avoid at any stage within the absorption unit the presence of an ignitable composition.

There is a considerable drawback associated with this procedure. Due to the addition of inert gas, the total gas stream within the absorption unit is drastically increased. Thereby, the efficiency of the absorption is reduced and larger absorption units and larger amounts of absorbing solvents are necessary to achieve the desired separation of valuable organic components from the exit gas stream of a reaction zone.

Thus, the object of the present invention is to provide a process for the recovery of combustible compounds of a gas stream comprising combustible components and oxygen that does not have the above discussed disadvantages while ensuring safety of the overall process.

This object has been attained by a process for the recovery of combustible components of a gas stream comprising the combustible components and oxygen by selective absorption of the combustible components in a solvent, whereby during the absorption the gas phase is dispersed in a continuous liquid phase of the solvent.

The present inventors have surprisingly discovered that if the gas phase comprising combustible components and oxygen is dispersed in a continuous liquid phase of the solvent during absorption, even if due to the depletion of combustible components from the gas phase the oxygen concentration in the gas phase rises above the explosion limit, the gas phase still cannot be ignited within the absorption unit because the gas phase is finely dispersed in the continuous liquid phase of the solvent. Consequently, addition of inert gas to the gas phase prior to entering the absorption unit or within the absorption zone in the absorption unit is not necessary any longer. Thus, it is preferred that neither prior to entering the absorption unit nor within the absorption zone an inert gas is added. This way, the absorption process is very efficient since the gas phase contains less or no inert gas. Consequently, the dimensions of the absorption unit can be reduced, thereby saving investment costs, and a reduced volume of the absorption fluid, i.e. the solvent, can be used, with the effect that the amount of solvent to be recycled or processed in working-up stages can be considerably reduced, thereby improving the overall economics of the process.

But although no inert gas is added prior to entering the absorption zone or in the absorption zone, safety of the process is ensured since the gas phase, although the oxygen content may be above the explosion limit, cannot be ignited.

The absorption unit is run as a bubble column.

According to a preferred embodiment, the gas bubbles dispersed in the continuous phase of the absorption solvent have a diameter of 10 millimeters or less, preferably 2-10 millimeters, most preferred 5 millimeters at the most.

According to a specifically preferred embodiment of the present invention, the gas stream is introduced into the absorption unit at a lower section of the absorption unit, and the liquid solvent phase enters the absorption unit at a position upwards with respect to the location the gas stream enters the absorption unit, and the liquid solvent phase exits the absorption unit at a position below the entry of the gas stream into the absorption unit. Thereby, it is achieved that the gas stream and the solvent pass through the absorption unit in a countercurrent manner. The absorption unit is run as bubble column.

The flow rate of the dispersed gas phase and of the continuous liquid solvent phase can be varied in wide ranges as long as the requirement that the liquid phase is continuous and the gas phase is dispersed is maintained. The flow rate per cross-section for the gas phase is preferably 10-100 m³/m²h, more preferred 20-60 m³/m²h and the flow rate per cross-section for the liquid phase is preferably 50 - 200 m³/m²h, more preferred 100 - 150 m³/m²h.

According to a preferred embodiment of the present invention, the gas stream is introduced into the absorption unit with a system of ring nozzles to finely disperse the gas phase in the continuous liquid phase. The dimensions of the absorption unit as well as the flow conditions of the gas phase and the continuous solvent phase are selected to provide gas bubbles dispersed in the continuous liquid phase having a diameter of 10 millimeters or less, preferably 2 - 10 millimeters, more preferred 5 millimeters at the most. Several measures can be taken singly or in combination to control the above defined bubbles size. For example the cross-section of the orifices of the ring nozzles can be selected to be within the range of 0.2 - 2 mm, and/or sieve trays can be positioned within the absorption unit in defined spacing having an orifice cross-section of 0.2 - 2 mm and/or the flow rate of the liquid phase and the gas phase are adjusted as defined above. If sieve tray are used the ratio of the free cross-section of the sieve trays to the cross-section of the absorption unit is preferably adjusted to ensure a flow rate of the gas phase through the perforated plates of 0.5-2 m/s.

The absorption unit that can be used in the process of the present invention may comprise heat exchange means and/or gas dispersing means. Heat exchange means can be useful to control the temperature in the absorption unit, especially to remove the heat of absorption. Gas dispersing means may be present to improve gas dispersion and the mass transfer between gas phase and continuous solvent phase. Preferably sieve trays and particularly preferably sieve trays with downcorners are used as gas dispersing means.

For safety reasons, it is preferred to introduce inert gas into the headspace above the liquid level within the absorption unit. Thereby, the gas stream exiting the liquid solvent phase is diluted to the extent that the oxygen concentration is below the explosion limit. Since the gas phase after exiting the liquid solvent phase is no longer finely dispersed, the gas mixture will become ignitable in case the oxygen concentration due to depletion of combustible components during the absorption is above the explosion limit.

Any inert gas is suitable that has an oxygen content of less than 10 volume per cent and does not form combustible mixtures with oxygen. Preferred inert gas is nitrogen, carbon dioxide or water vapor or mixtures thereof, as may be obtained by usual combustion processes.

According to a preferred embodiment of the present invention no inert gas or only very limited amounts of inert gas are introduced into the gas stream prior to entering the absorption unit or into the absorption zone within the absorption unit to achieve the reduction of absorption unit dimensions and volume of solvent. A suitable upper limit of the ratio of inert gas introduced into the gas stream prior to absorption or into the absorption zone is to oxygen present in the gas stream is 5 : 1. But it is preferred to introduce no inert gas into the absorption zone during the absorption process.

According to an alternative embodiment, the volume of the headspace in the absorption unit above the liquid level is reduced by displacers and the absorption unit is equipped with pressure release means and a flame barrier in the gas exit line. In that case, the amount of inert gas introduced into the headspace can be considerably reduced while ensuring sufficient safety for the absorption process.

As continuous phase within the absorption unit, any liquid can be used that dissolves the combustible components that shall be recovered better than oxygen and that allows easy separation of the recovered components. Thereby, usual organic solvents like alcohols, aromatic and aliphatic hydrocarbons or ketones can be used. It is preferred to use a solvent that is also used in the process the gas stream is derived from prior to the absorption. This is advantageous since then the solvent stream can be either directly recycled into the reaction zone or can be passed to a working-up stage downstream of the reaction stage.

In the process of the present invention, any gas stream from a reaction step wherein oxygen is generated and that contains combustible materials can be processed to recover valuable combustible components. It is particularly preferred that the gas stream is the gaseous effluent of an oxidation process using peroxide compounds. The process of the present invention is especially preferred to recover olefin, and olefin oxide from the gaseous effluent from the epoxidation of an olefin with hydrogen peroxide.

Therefore, the present invention also refers to a process for the epoxidation of propene with hydrogen peroxide in an alcoholic solvent in the presence of a catalyst, wherein a gas stream comprising unreacted propene, propene oxide, and oxygen from the decomposition of the hydrogen peroxide is separated from the reaction mixture and the combustible components in said gas stream are recovered using the process as described above.

According to a particularly preferred embodiment, the same alcoholic solvent as in the epoxidation step is also used in the recovery step. The preferred alcohol is methanol.

The pressure within the absorption unit in the recovery process according to the present invention can be varied within wide ranges and can preferably be between atmospheric pressure and 50 bar, in case of absorption of propene and propene oxide the pressure is preferably within a range of 4-30 bar, more preferred between 10-25 bar.

The present invention will now be discussed in more detail with respect to the attached figure.

Figure 1 shows a suitable absorption unit for the present process in cross-section.

Figure 2 shows a preferred absorption unit according to the present invention in cross-section.

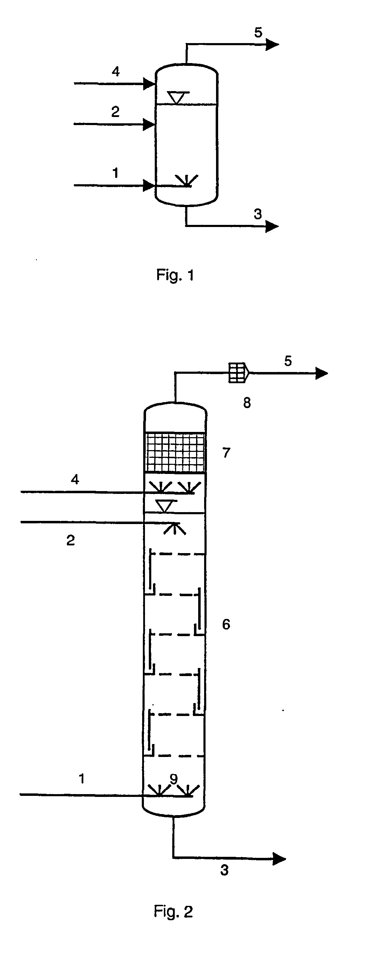

Referring to Figure 1 a tubular absorption unit is shown, wherein the gas stream 1 comprising combustible components and oxygen is introduced into the absorption unit through an inlet that is positioned near the lower end of the absorption unit. The inlet for the solvent stream 2 that forms the continuous liquid phase within the absorption zone is positioned closer to the upper end of the absorption unit. The outlet for the solvent 3 loaded with the absorbed combustible components is located at the bottom of the absorption unit. At the upper end of the absorption unit, an inlet port is positioned to allow introduction of an inert gas 4 in order to dilute the gas prior to exiting the absorption unit to an oxygen concentration below the explosion limit. The exit gas stream 5 comprising oxygen, small amounts of combustible components, and optionally inert gas exits the absorption unit through an outlet positioned at the top of the absorption unit.

Referring to figure 2 according to a preferred embodiment the gas stream 1 is introduced into the absorption unit through a system of ring nozzles 9 to ensure efficient dispersion of the gas phase within the continuous liquid phase. Within the absorption zone in the absorption unit, sieve trays 6 with downcomers are mounted to improve the mass transfer between the continuous phase and the dispersed gas to improve the absorption. The volume of the headspace above the fluid level at the upper end of the absorption unit is reduced by incorporation of displacers 7 and a flame barrier 8 is positioned in the gas exit line.

The present invention will now be explained in more detail with reference to examples.

### Example 1

A tube having a diameter of 76 millimeters and a length of 1,000 millimeters was filled with benzene and the temperature was maintained at 70°C. At the lower end, pure oxygen was injected at 4 bar pressure at a flow rate of 200 liters per hour. The portion of the gas phase within the tubular absorption unit was 5 volume per cent as measured by the increase of the height of the liquid level. Due to the volatility of benzene, the uprising dispersed gas phase will be loaded with benzene and immediately reaches an ignitable composition. A glow wire was immersed 10 centimeters below the liquid level, and it was tried to ignite the gas phase by applying an electrical pulse of 500 Watts. But no ignition of the dispersed gas phase could be observed.

### Example 2

Example 1 was repeated with an oxygen flow of 170 liters per hour, while the portion of the dispersed gas phase within the liquid phase was 25 volume per cent. But also in this experiment, no ignition of the dispersed gas phase could be observed.

The examples show that a gas phase containing a combustible material and oxygen in a ratio that is within the explosion range cannot be ignited as long as the gas phase is finely dispersed within a continuous liquid phase.

## Claims

1. Process for the recovery of combustible components of a gas stream comprising the combustible components and oxygen by selective absorption of the combustible components in a solvent,
**characterized in that**
during the absorption the gas phase is dispersed in a continuous liquid phase of the solvent in an absorption unit that is run as a bubble column thereby using an organic solvent.

2. The process of claim 1, **characterized in that**
the process is a continuous process.

3. The process of any of the preceding claims, **characterized in that**
the gas bubbles dispersed in the continuous phase have a diameter of 10 mm or less, preferably of 5 mm at the most.

4. The process of any of the preceding claims, **characterized in that**
the gas stream is introduced into an absorption unit at a lower section of the absorption unit and the liquid solvent phase enters the absorption unit at a position upwards with respect to the location the gas stream enters the absorption unit, whereby the gas stream and the solvent pass through the absorption unit counter-currently and the liquid solvent phase exits the absorption unit at a position below the entry of the gas stream into the absorption unit.

5. The process of any of the preceding claims, **characterized in that**
the volume of the head space above the liquid level of the continuous phase is reduced by displacers.

6. The process of any of claims 1, 4 and 5, **characterized in that**
the gas stream exiting the liquid solvent phase is diluted with an inert gas to the extent that the oxygen concentration is below the explosion limit.

7. The process of claim 6, **characterized in that**
the inert gas is selected from nitrogen, water vapor, carbon dioxide or mixtures thereof.

8. The process of any of claims 1, 4-7, **characterized in that**
the gas stream is introduced into the absorption unit through a system of ring nozzles.

9. The process of any of claims 1, 4-8, **characterized in that**
the flow conditions within the absorption unit are selected to provide gas bubbles dispersed in the continuous liquid phase having a diameter of 10 mm or less, preferably of 5 mm at the most.

10. The process of any of claims 1, 4-9, **characterized in that**
the absorption unit comprises heat exchange means and/or gas dispersing means.

11. The process of claim 10, **characterized in that**
sieve trays are positioned within the absorption unit.

12. The process of any of the preceding claims, **characterized in that**
the gas stream is the gaseous effluent of an oxidation process using peroxide compounds.

13. The process of any of the preceding claims, **characterized in that**
the solvent is selected from alcohols, aliphatic and aromatic hydrocarbons and ketones.

14. A process for the epoxidation of propene with hydrogen peroxide in an alcoholic solvent in presence of a catalyst, wherein a gas stream comprising unreacted propene, propene oxide and oxygen from the decomposition of the hydrogen peroxide is separated from the reaction mixture and the combustible components in said gas stream are recovered using a process according to any of claims 1-13.

15. The process of claim 14, wherein in the recovery step the same alcoholic solvent as in the epoxidation step is used.

16. The process of claim 15, wherein the solvent is methanol.

17. The process of any of claims 15 and 16, wherein the liquid solvent phase comprising the recovered combustible components is either recycled to the epoxidation step or is passed to a working up stage downstream from the epoxidation step.

## Patentansprüche

1. Verfahren zur Rückgewinnung von brennbaren Komponenten aus einem Gasstrom, der die brennbaren Komponenten und Sauerstoff enthält, durch selektive Absorption der brennbaren Komponenten in einem Lösungsmittel,
**dadurch gekennzeichnet, dass**
die Gasphase während der Absorption in einer kontinuierlichen flüssigen Phase des Lösungsmittels in einer Absorptionseinheit, die als Blasensäule betrieben wird, dispergiert ist und dabei ein organisches Lösungsmittel verwendet wird.

2. Verfahren nach Anspruch 1,
**dadurch gekennzeichnet, dass**
das Verfahren ein kontinuierliches Verfahren ist.

3. Verfahren nach einem der vorstehenden Ansprüche,
**dadurch gekennzeichnet, dass**
die Gasblasen, die in der kontinuierlichen Phase dispergiert sind, einen Durchmesser von 10 mm oder weniger, vorzugsweise höchstens 5 mm, aufweisen.

4. Verfahren nach einem der vorstehenden Ansprüche,
**dadurch gekennzeichnet, dass**
der Gasstrom in eine Absorptionseinheit in einem unteren Bereich der Absorptionseinheit eingebracht wird und die flüssige Lösungsmittelphase in die Absorptionseinheit an einer Stelle oberhalb bezüglich des Orts, an dem der Gasstrom in die Absorptionseinheit eintritt, eintritt, wobei der Gasstrom und das Lösungsmittel die Absorptionseinheit im Gegenstrom durchlaufen und die flüssige Lösungsmittelphase die Absorptionseinheit an einer Stelle unterhalb des Eintritts des Gasstroms in die Absorptionseinheit verlässt.

5. Verfahren nach einem der vorstehenden Ansprüche,
**dadurch gekennzeichnet, dass**
das Volumen des Kopfraums oberhalb des Flüssigkeitsniveaus der kontinuierlichen Phase durch Verdrängerkörper reduziert ist.

6. Verfahren nach einem der Ansprüche 1, 4 und 5,
**dadurch gekennzeichnet, dass**
der Gasstrom, der die flüssige Lösungsmittelphase verlässt, mit einem Inertgas in dem Maße verdünnt wird, dass die Sauerstoffkonzentration unterhalb der Explosionsgrenze ist.

7. Verfahren nach Anspruch 6,
**dadurch gekennzeichnet, dass**
das Inertgas ausgewählt ist aus Stickstoff, Wasserdampf, Kohlendioxid oder Mischungen davon.

8. Verfahren nach einem der Ansprüche 1 und 4-7,
**dadurch gekennzeichnet, dass**
der Gasstrom in die Absorptionseinheit durch ein System von Ringdüsen eingebracht wird.

9. Verfahren nach einem der Ansprüche 1 und 4-8,
**dadurch gekennzeichnet, dass**
die Strömungsbedingungen innerhalb der Absorptionseinheit so ausgewählt sind, um Gasblasen zur Verfügung zu stellen, die in der kontinuierlichen flüssigen Phase dispergiert sind und einen Durchmesser von 10 mm oder weniger, vorzugsweise von höchstens 5 mm, aufweisen.

10. Verfahren nach einem der Ansprüche 1 und 4-9,
**dadurch gekennzeichnet, dass**
die Absorptionseinheit Wärmeaustauschmittel und/oder Gasdispergierungsmittel enthält.

11. Verfahren nach Anspruch 10,
**dadurch gekennzeichnet, dass**
Siebböden innerhalb der Absorptionseinheit angeordnet sind.

12. Verfahren nach einem der vorstehenden Ansprüche,
**dadurch gekennzeichnet, dass**
der Gasstrom ein gasförmiger Austrittstrom aus einem Oxidationsprozess ist, bei dem Peroxidverbindungen verwendet werden.

13. Verfahren nach einem der vorstehenden Ansprüche,
**dadurch gekennzeichnet, dass**
das Lösungsmittel ausgewählt ist aus Alkoholen, aliphatischen und aromatischen Kohlenwasserstoffen und Ketonen.

14. Verfahren zur Epoxidierung von Propen mit Wasserstoffperoxid in einem alkoholischen Lösungsmittel in Gegenwart eines Katalysators, wobei ein Gasstrom, der nichtumgesetztes Propen, Propenoxid und Sauerstoff aus der Zersetzung des Wasserstoffperoxids enthält, aus der Reaktionsmischung abgetrennt wird und die brennbaren Komponenten dieses Gasstroms unter Verwendung eines Verfahrens nach einem der Ansprüche 1-13 zurückgewonnen werden.

15. Verfahren nach Anspruch 14, wobei in dem Rückgewinnungsschritt dasselbe alkoholische Lösungsmittel wie in dem Epoxidierungsschritt verwendet wird.

16. Verfahren nach Anspruch 15, wobei das Lösungsmittel Methanol ist.

17. Verfahren nach einem der Ansprüche 15 und 16, wobei die flüssige Lösungsmittelphase, die die zurückgewonnenen brennbaren Komponenten enthält, entweder in den Epoxidierungsschritt zurückgeführt wird oder zu einer Aufarbeitungsstufe nach dem Epoxidierungsschritt zugeführt wird.

## Revendications

1. Procédé pour la récupération de composants combustibles dans un courant gazeux comprenant les composants combustibles et de l'oxygène, par absorption sélective des composants combustibles dans un solvant,
**caractérisé en ce que**
pendant l'absorption, la phase gazeuse est dispersée dans une phase liquide continue du solvant dans une unité d'absorption qui fonctionne en tant que colonne à bulles, en utilisant de cette manière un solvant organique.

2. Procédé selon la revendication 1,
**caractérisé en ce que**
le procédé est un procédé continu.

3. Procédé selon l'une quelconque des revendications précédentes,
**caractérisé en ce que**
les bulles de gaz dispersées dans la phase continue ont un diamètre de 10 mm ou inférieur, de préférence de 5 mm au plus.

4. Procédé selon l'une quelconque des revendications précédentes,
**caractérisé en ce que**
le courant gazeux est introduit dans une unité d'absorption au niveau d'un segment inférieur de l'unité d'absorption, et la phase solvant liquide entre dans l'unité d'absorption dans une position vers le haut par rapport à l'emplacement où le courant gazeux entre dans l'unité d'absorption, le courant gazeux et le solvant traversant de cette manière l'unité d'absorption à contre-courant, et la phase solvant liquide sort de l'unité d'absorption dans une position au-dessous de l'entrée du courant gazeux dans l'unité d'absorption.

5. Procédé selon l'une quelconque des revendications précédentes,
**caractérisé en ce que**
le volume de l'espace vide au-dessus du niveau de liquide de la phase continue est réduit par des agitateurs.

6. Procédé selon l'une quelconque des revendications 1, 4 et 5,
**caractérisé en ce que**
le courant gazeux sortant de la phase solvant liquide est dilué avec un gaz inerte jusqu'à ce que la concentration d'oxygène soit inférieure à la limite d'explosion.

7. Procédé selon la revendication 6,
**caractérisé en ce que**
le gaz inerte est choisi parmi l'azote, la vapeur d'eau, le dioxyde de carbone ou des mélanges de ceux-ci.

8. Procédé selon l'une quelconque des revendications 1, 4 à 7,
**caractérisé en ce que**
le courant gazeux est introduit dans l'unité d'absorption par un système annulaire de tuyères.

9. Procédé selon l'une quelconque des revendications 1, 4 à 8,
**caractérisé en ce que**
les conditions d'écoulement à l'intérieur de l'unité d'absorption sont choisies pour fournir des bulles de gaz dispersées dans la phase liquide continue ayant un diamètre de 10 mm ou moins, de préférence de 5 mm au plus.

10. Procédé selon l'une quelconque des revendications 1, 4 à 9,
**caractérisé en ce que**
l'unité d'absorption comprend des moyens échangeurs de chaleur et/ ou des moyens disperseurs de gaz.

11. Procédé selon la revendication 10,
**caractérisé en ce que**
des plateaux de criblage sont placés à l'intérieur de l'unité d'absorption.

12. Procédé selon l'une quelconque des revendications précédentes,
**caractérisé en ce que**
le courant gazeux est l'effluent gazeux d'un processus d'oxydation utilisant des composés peroxydiques.

13. Procédé selon l'une quelconque des revendications précédentes,
**caractérisé en ce que**
le solvant est choisi parmi les alcools, les hydrocarbures aliphatiques et aromatiques et les cétones.

14. Procédé pour l'époxydation du propène avec du peroxyde d'hydrogène dans un solvant alcoolique en présence d'un catalyseur, selon lequel un courant gazeux comprenant du propène n'ayant pas réagi, de l'oxyde de propène et de l'oxygène provenant de la décomposition du peroxyde d'hydrogène, est séparé du mélange réactionnel, et des composants combustibles dans le courant gazeux sont récupérés en utilisant un procédé selon l'une quelconque des revendications 1 à 13.

15. Procédé selon la revendication 14,
selon lequel à l'étape de récupération, on utilise le même solvant alcoolique qu'à l'étape d'époxydation.

16. Procédé selon la revendication 15,
selon lequel le solvant est le méthanol.

17. Procédé selon l'une quelconque des revendications 15 et 16,
selon lequel la phase solvant liquide comprenant les composants combustibles récupérés est soit recyclée à l'étape d'époxydation, soit passée à une étape d'élaboration en aval de l'étape d'époxydation.
